# EUROPEAN PATENT SPECIFICATION

(11) **EP 3 183 366 B1**
(45) Date of publication and mention of the grant of the patent: **19.10.2022**
(21) Application number: 15833890.5
(22) Date of filing: 18.08.2015
(51) Int. Cl.: C12Q 1/68, C12Q 1/37, A61K 39/00, C07K 16/40, C12P 21/08

(54) **SYSTEMS AND METHODS OF DETECTING SOLID TUMOR CANCER**
SYSTEME UND VERFAHREN FÜR DEN NACHWEIS SOLIDER MALIGNER TUMOREN
SYSTÈMES ET PROCÉDÉS DE DÉTECTION D'UN CANCER À TUMEUR SOLIDE

(30) Priority: 18.08.2014 US 201462038721 P
(43) Date of publication of application: 28.06.2017
(73) Proprietor: Adrastia Biotech Corporation, Lafayette, CA 94549 (US)
(72) Inventor: COURTNEY, Angela, Inglewood, CA 90305 (US)
(74) Representative: Viering, Jentschura & Partner mbB Patent- und Rechtsanwälte
(86) International application number: PCT/US2015/045739
(87) International publication number: WO 2016/028805

(56) References cited:
- WO-A1-2013/173485
- WO-A2-2008/134526
- WO-A2-2014/107599
- US-A1- 2008 287 665
- US-A1- 2009 047 216
- US-A1- 2010 184 049
- US-A1- 2011 091 377
- US-A1- 2014 105 824
- US-A1- 2014 105 824
- MOHAMED M. HAFEZ ET AL: "MicroRNAs and Metastasis-related Gene Expression in Egyptian Breast Cancer Patients", ASIAN PACIFIC JOURNAL OF CANCER PREVENTION, vol. 13, no. 2, 29 February 2012 (2012-02-29), pages 591-598, XP055454818, TH ISSN: 1513-7368, DOI: 10.7314/APJCP.2012.13.2.591
- Yoshiki Kajiwara ET AL: "Heterogeneity of Metalloproteinase Expression in Colorectal Cancer - Relation of Molecular Findings to Basic Morphology", anticancer research, 1 May 2011 (2011-05-01), pages 1567-1576, XP055454653, Retrieved from the Internet: URL:http://ar.iiarjournals.org/content/31/ 5/1567.full.pdf [retrieved on 2018-02-27]
- MURPHY. ET AL.: 'The ADAMs: signalling scissors in the tumour microenvironment.' NAT REV CANCER. vol. 8, no. 12, December 2008, pages 929 - 941, XP009500254
- ROY ET AL.: 'ADAM 12 cleaves extracellular matrix proteins and correlates with cancer status and stage.' J BIOL CHEM. vol. 279, no. 49, 03 December 2004, pages 51323 - 51330, XP002321631

## Description

### BACKGROUND OF THE INVENTION

### FIELD OF THE INVENTION

The present disclosure relates generally to diagnostic techniques for breast cancer and, more specifically, to biomarkers of breast cancer in a biological sample, such as urine.

### BACKGROUND INFORMATION

The goal of cancer treatment is to find, identify and remove a solid state cancer mass before it has metastasized to distant organs to cause death. The earlier a cancer mass is detected in a patient, the better the outcome in the long term. Finding cancer in a patient before it causes a physical illness is difficult. The microenvironment around a solid tumor mass is unique and in a cross talk loop between the cancer cells and cells in the surrounding microenvironment tissues. This crosstalk is mediated through a variety of proteins some of which are present in the urine and have increased expression levels as the solid state tumor grows.

The tumor microenvironment has common cross talk proteins with the microenvironment regardless of the receptors and genes present on and in the tumor itself (Kanakis, D., et al., Dis Markers, 2013. 34(2): p. 81-91; Gerhards, S., et al., Urology, 2001. 57(4): p. 675-9). For example, although breast cancer has a myriad of different receptor types, such as HER2, Triple Negative, Estrogen positive and subtypes based upon genomic expression, the receptors all have in common the tumor microenvironment.

The tumor microenvironment is elevated in common crosstalk proteins, which allow the tumor to grow, expand and reside in the microenvironment (Pories, S.E., et al., Cancer Epidemiol Biomarkers Prev, 2008. 17(5): p. 1034-42). It is these cross talk proteins of the tumor microenvironment that are detected by the methods disclosed herein in order to provide evidence of the presence of the solid state cancer mass in the patient/subject (Roy, R., et al., J Biol Chem, 2004. 279(49): p. 51323-30). Because these cross talk biomarker proteins are present in the urine, they can be monitored for levels in a non-invasive manner to scan for the early presence and detection of a cancer solid state mass in any individual as often as necessary.

The international patent application WO 2008/134526 A2 discloses a method for detecting or diagnosing a urogenital related cancer in a subject, comprising detecting the presence of and/or quantifying the level of at least one specific biomarker protein or a nucleic acid encoding a specific biomarker in a biological sample from the substrate, wherein the presence of the biomarker, or a level or concentration of the biomarker above a pre-determined threshold is indicative of cancer in the subject.

The international patent application WO 2013/173485 A1 discloses a method of screening for bladder cancer, the method comprising: establishing a cutoff level for a protein marker selected from the group consisting of MMP2 and MMP9 to provide a predetermined sensitivity for an assay, wherein the protein cutoff level is indicative of the absence of cancer; establishing a cutoff level for two or more nucleic acid markers selected from the group consisting of FGFR3, TWISTI, and NID2, wherein said nucleic acid cutoff levels increase the specificity of the assay without decreasing the sensitivity of the assay, and wherein said nucleic acid cutoff levels are indicative of the absence of cancer; conducting the assay in a tissue or bodily fluid sample to determine a level of said protein marker and a level of said nucleic acid markers in the sample; and identifying said sample as positive for bladder cancer if the level of said protein marker and said nucleic acid markers are greater than their respective cutoff levels.

The publication from Hafez et al. (Hafez, M. M., et al. Asian Pacific Journal Of Cancer Prevention, 2012. 13(2): p. 591-598) describes the study of miRNAs in Egyptian breast cancer. Furthermore, the regulation of MMP2, MMP9 and VEGF genes is examined.

The publication from Kajiwara et al. (Kajiwara, Y., et al. Anticancer research, 2011. 31:1567-1576) describes the correlation between morphological features and the mRNA expression of ADAMs and MMPs in colorectal cancer.

There exists a need for methods that enable early detection of a cancer mass, thereby allowing early treatment, increased routine scanning for the markers as part of a health examination and increased survival.

### SUMMARY OF THE INVENTION

The present invention is defined by the appended claims. The present invention is based on the seminal discovery that certain biomarkers present in a biological sample, such as urine, allow for early detection of solid state cancer mass presence.

In one aspect, the present disclosure provides a method of detecting breast cancer in a subject, comprising, determining, in the sample from the subject, the expression level of biomarkers of solid tumor microenvironment potential or solid state tumor mass potential consisting of metalloproteinase 9 (MMP9), metalloproteinase 2 (MMP2), nuclear factor kappa-light-chain-enhancer of activated B cells (NF-κB), and uromodulin, as compared to a control standard or the expression of the biomarkers in a control sample, wherein differential expression of the biomarkers in the sample as compared to the control standard or the expression of the biomarkers in a control sample indicates presence of breast cancer in the subject.

In certain embodiments, the expression levels measured are protein, mRNA or microRNA expression levels. In other embodiments, the expression levels are determined by ELISA. In yet other embodiment, the subject is a human. In one embodiment, the protein, mRNA or microRNA expression level measurements are quantitative. In another embodiment, the biological sample is a urine sample.

In various embodiments, all of the biomarkers, consisting of metalloproteinase 9 (MMP9), metalloproteinase 2 (MMP2), uromodulin, and nuclear factor kappa-light-chain-enhancer of activated B cells (NF-κB) exhibit increased expression in a tumor sample as compared to control.

Certain aspects of the presently disclosed subject matter having been stated hereinabove, which are addressed in whole or in part by the presently disclosed subject matter, other aspects will become evident as the description proceeds when taken in connection with the accompanying Examples and Figures as best described herein below.

### BRIEF DESCRIPTION OF THE DRAWINGS

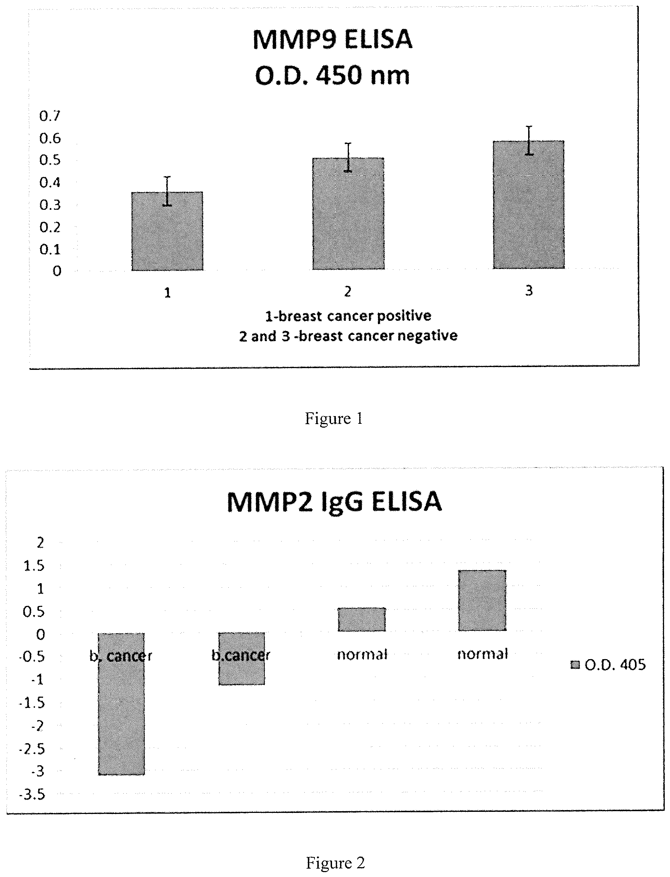

Figure 1 is a graphical display representing data relating to embodiments of the disclosure. The graph shows experimental data generated by the inventor of MMP9 detection using ELISA in breast cancer positive (1) and breast cancer negative (2 and 3) samples. MMP9 is shown to be differentially expressed (decreased expression as compared to control) in breast cancer.
Figure 2 is a graphical display representing data relating to embodiments of the disclosure. The graph shows experimental data generated by the inventor of MMP2 detection using ELISA in breast cancer positive and normal control samples. MMP2 is shown to be differentially expressed (decreased expression as compared to control) in breast cancer.

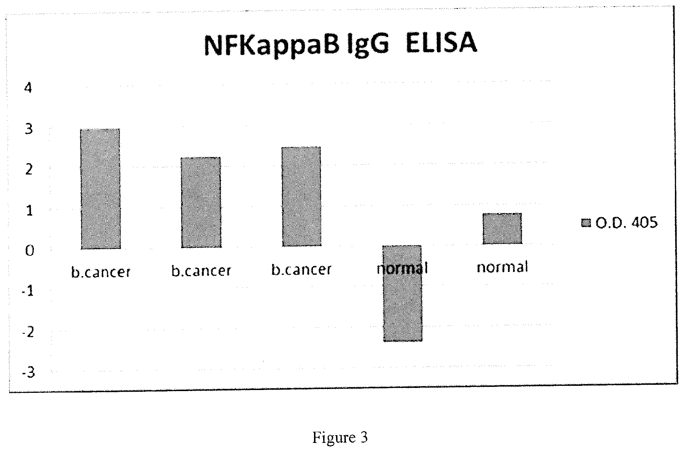

Figure 3 is a graphical display representing data relating to embodiments of the disclosure. The graph shows experimental data generated by the inventor of NF-κB detection using ELISA in breast cancer positive and normal control samples. NF-κB is shown to be differentially expressed (increased expression as compared to control) in breast cancer.

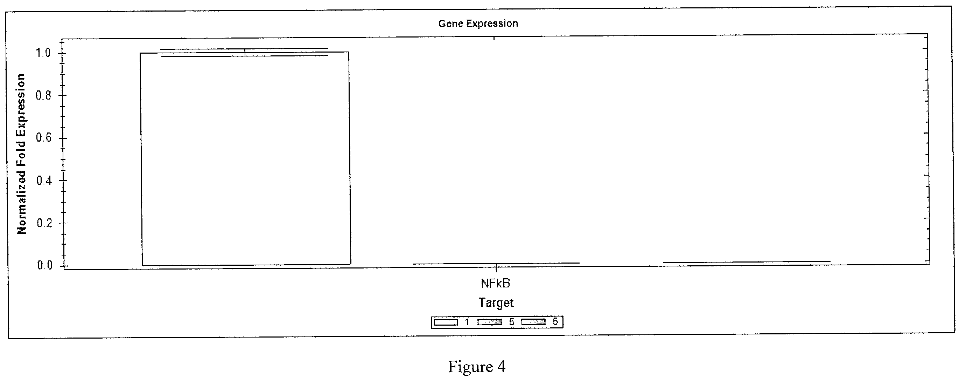

Figure 4 is a graphical display representing data relating to embodiments of the disclosure. The graph shows experimental data generated by the inventor of NF-κB detection using a PCR based assay (TAQMAN^{®}) in breast cancer positive (1; left panel) and breast cancer negative (5 and 6; middle and right panel) samples.

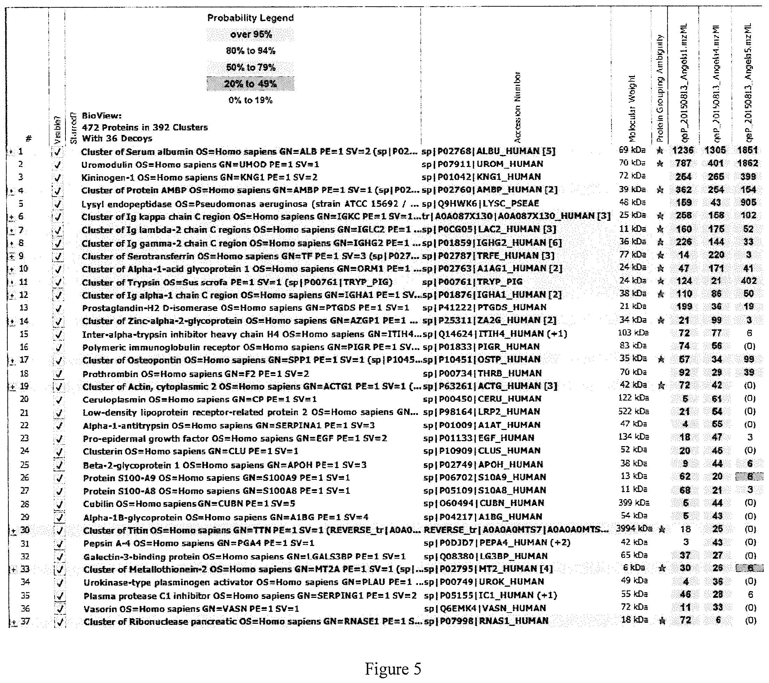

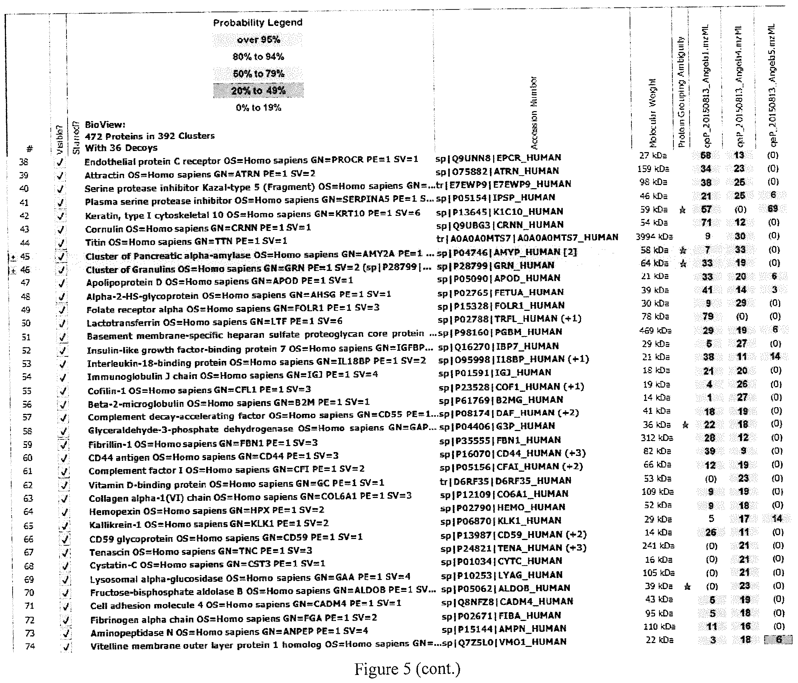

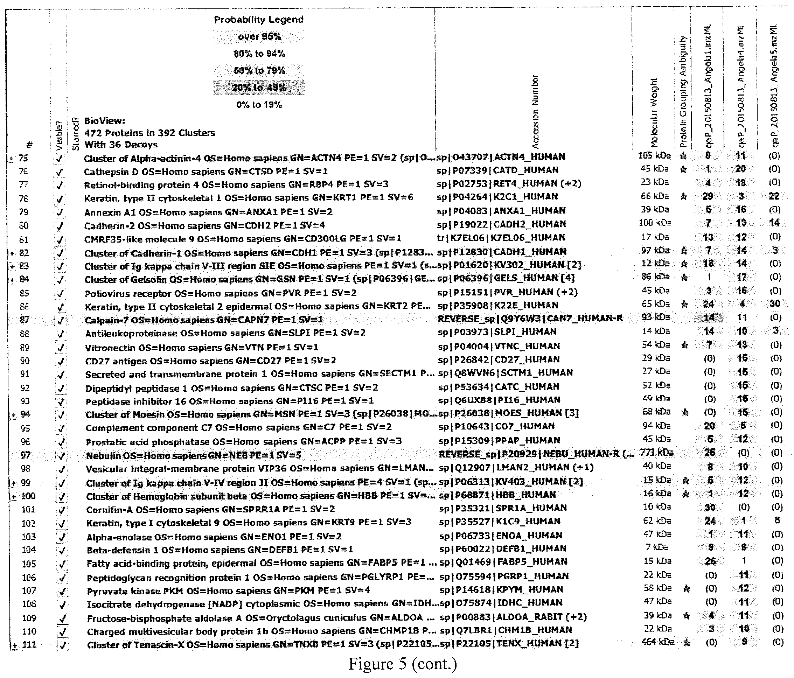

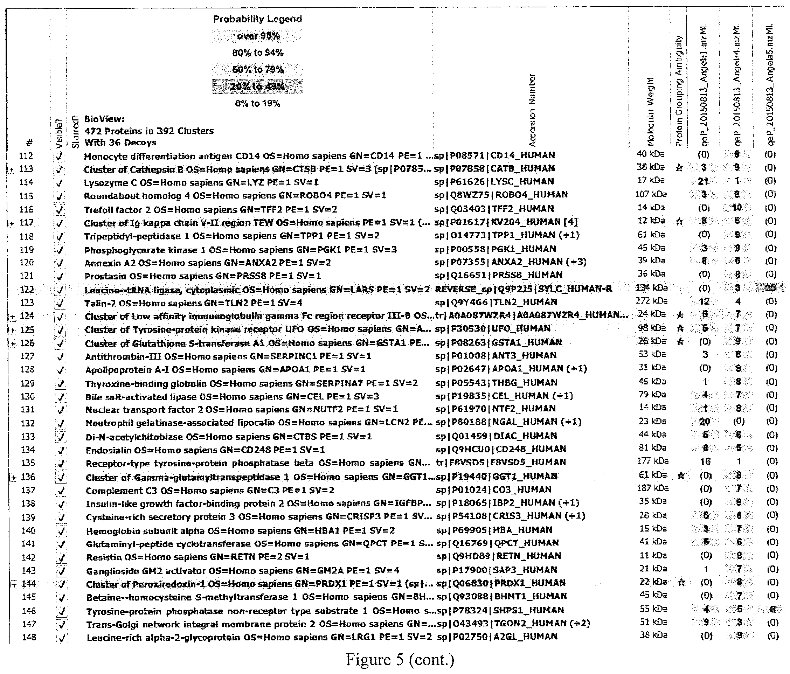

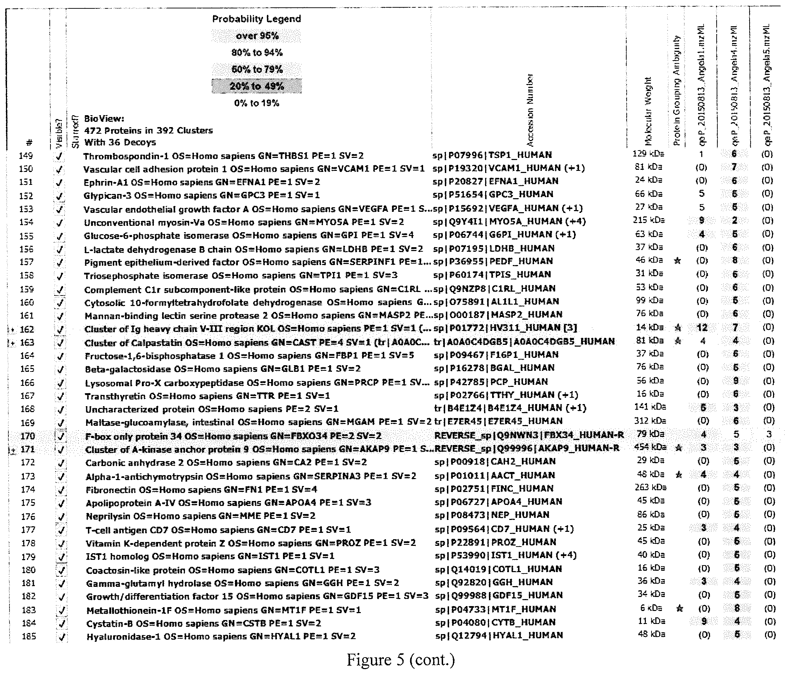

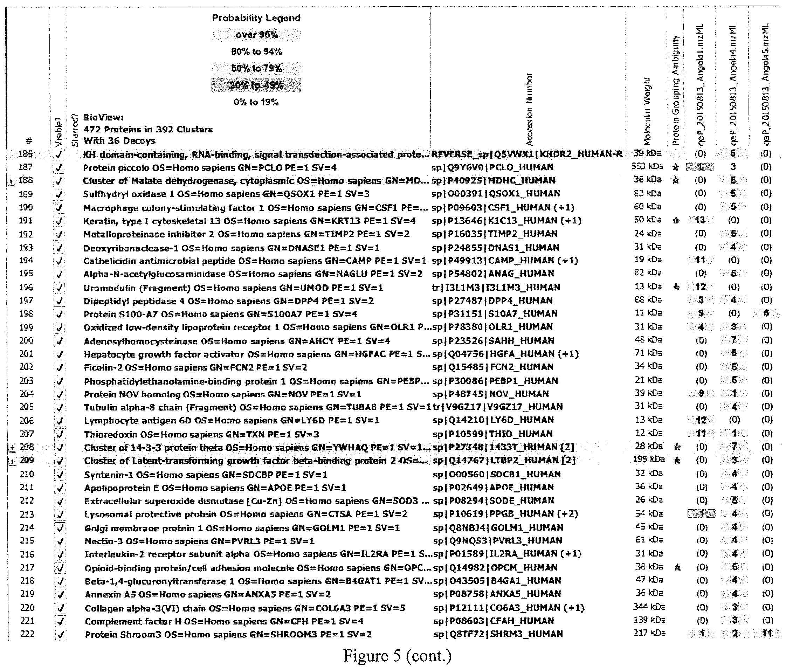

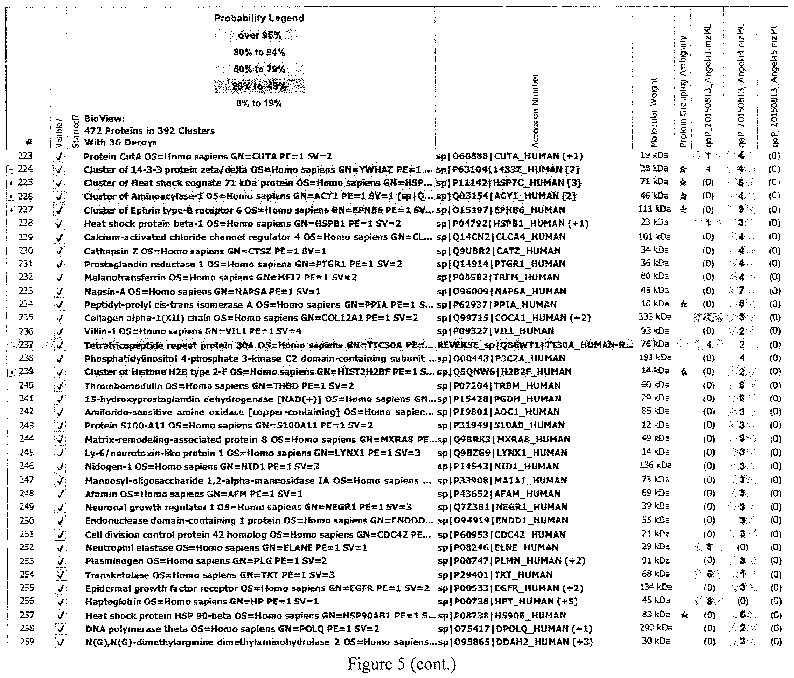

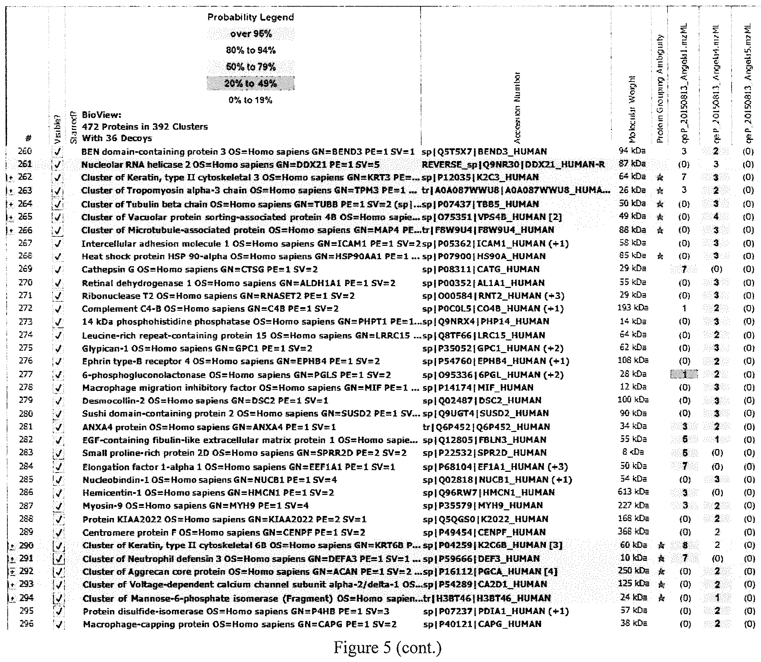

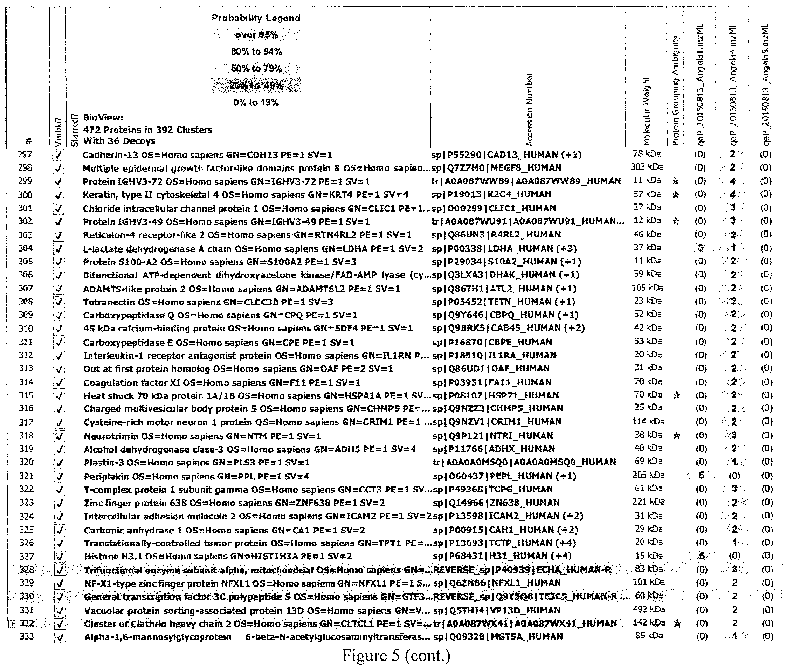

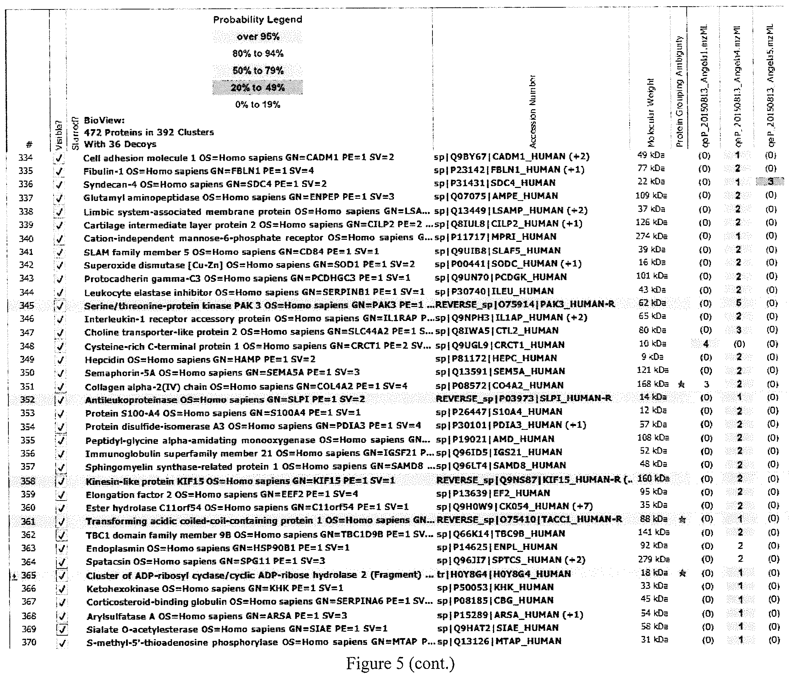

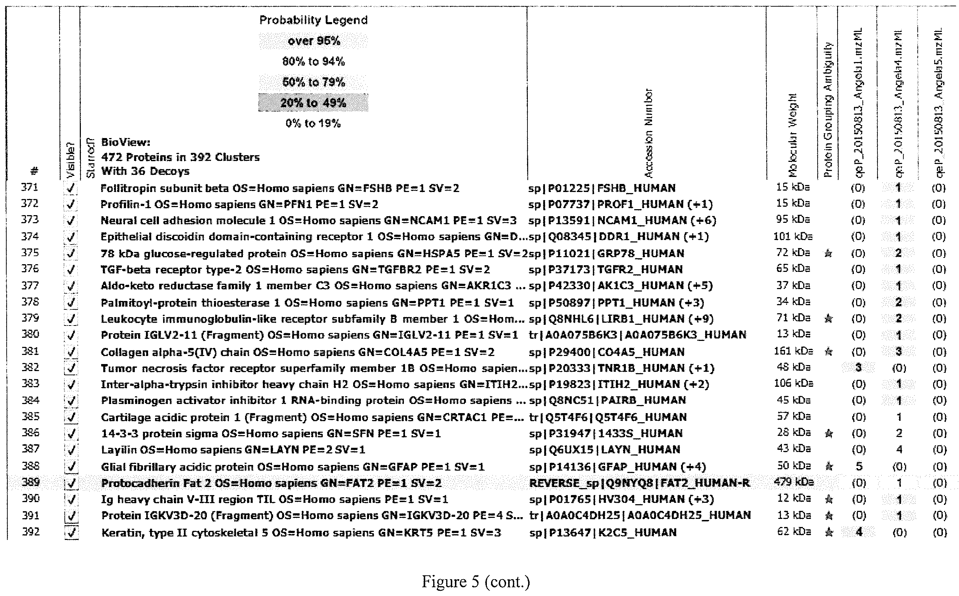

Figure 5 is a graphical display representing data relating to embodiments of the disclosure generated by the inventor. The table sets forth protein expression levels of various proteins in breast cancer positive samples (second and third columns from the right) and a breast cancer negative sample (first column from the right).

### DETAILED DESCRIPTION OF THE INVENTION

The present invention is defined in the appended claims. Disclosed herein are biomarkers of solid tumor presence, characterized at least in part by altered (e.g., increased or decreased) expression of one or more genes including ADAM metallopeptidase domain 12 (ADAM12), metalloproteinase 9 (MMP9), metalloproteinase 12 (MMP12), metalloproteinase 2 (MMP2), IgG, uromodulin and nuclear factor kappa-light-chain-enhancer of activated B cells (NF-κB) in subjects afflicted with the disease. The disclosed panel of biomarkers is useful to screen subject for cancer. Methods and compositions (including kits) that embody this discovery are described herein.

Before the present compositions and methods are described in further detail, it is to be understood that this invention is not limited to particular compositions, methods, and experimental conditions described, as such compositions, methods, and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

It is expected that the panel identification of the outlined matrix of protein levels will be used as an initial screening test in one aspect. As part of a routine health examination, a patient sample, *i.e.,* a urine sample, will be screened for the protein levels as a general gradation of presence (example -, +, ++, +++). Therefore, in one convenient embodiment, the test is formatted as a urine test strip (e.g., as a standalone strip, cassette or dipstick for laboratory use).

Preferably, patients that screen with elevated levels compared to clinical trial levels established as normal, are then retested in the clinical laboratory setting to determine the measured levels of the elevated proteins to obtain a numerical value. These levels are used as both a benchmark level to compare for future testing for rising levels and if elevated the levels are matched to validated tumor type and size. Identification of tumor masses before they are of a size large enough to be detected by current diagnostic technology is made possible by the invention. Detection may therefore be achieved when the tumor mass is too small to have metastasized (currently the size limit is 7mm) and the patient can be monitored for rising levels which may suggest metastasis has begun.

Once the levels reach those that have been validated to a tumor size that can be visualized with current diagnostic methods (such as PET Scan, ultrasound, mammography, and/or MRI). Confirmation of the diagnosis can be made visually and the appropriate treatment/removal performed.

Because tumor masses can be detected by the technology disclosed herein before they have reached the critical size to metastasize, tumors can be detected and treated/removed at the minimum size that they can be detected by current diagnostic technology. Patients can be routinely screened as often as deemed necessary due to the non-invasive nature of the test. The societal benefits are calculable: if the adult population, starting at age 20 for females and 40, for males is routinely screened cancer mortality, health care costs due to cancer treatment can be significantly decreased. Early detection and treatment at this level changes solid state cancer from an extensive life-threatening disease with debilitating treatment to an early intervention and removal for future generations.

Disclosed herein is a panel for detecting biomarkers of solid state cancer. In one embodiment, there is provided a panel for detecting biomarkers of solid state cancer, wherein the biomarkers comprise at least two of ADAM12, MMP9, NF-κB, MMP12, MMP2, uromodulin, IgG and Hyaluronic Acid (HA). The panel may further include one or more proteins or genes as set forth in Figure 5, especially those shown to exhibit increased expression in cancer.

Further disclosed is a method of determining solid state tumor potential. In one embodiment, the method comprises measuring expression levels in a sample of at least two biomarkers of solid state cancer mass potential, which are each independently ADAM12, MMP9, NF-κB, MMP12, MMP2, uromodulin, IgG, Hyaluronic Acid (HA) and comparing the expression levels to a control to determine solid state cancer mass potential. The method may further include one or more proteins or genes as set forth in Figure 5, especially those shown to exhibit increased expression in cancer.

Disclosed herein is a method for detecting a solid tumor cancer in a subject. The method includes: (a) obtaining a sample from the subject; and (b) determining, in the sample, the expression level of one or more biomarkers of solid tumor microenvironment potential or solid state tumor mass potential, the biomarker comprising ADAM metallopeptidase domain 12 (ADAM12), metalloproteinase 9 (MMP9), metalloproteinase 12 (MMP12), metalloproteinase 2 (MMP2), nuclear factor kappa-light-chain-enhancer of activated B cells (NF-κB), uromodulin, IgG or any combination thereof, as compared to a control standard or the expression of the biomarker in a control sample; wherein differential expression in the sample as compared to the control standard or the expression of the biomarker in a control sample indicates presents of a solid tumor cancer, thereby detecting solid tumor cancer in the subject. According to the disclosure, in embodiments, the method further includes detection of hyaluronic acid (HA). The disclosed method may further include one or more proteins or genes as set forth in Figure 5, especially those shown to exhibit increased expression in cancer.

In one aspect, the present invention provides a method of detecting breast cancer in a subject, comprising, determining, in a sample from the subject, the expression level of biomarkers of solid tumor microenvironment potential or solid state tumor mass potential consisting of metalloproteinase 9 (MMP9), metalloproteinase 2 (MMP2), nuclear factor kappa-light-chain-enhancer of activated B cells (NF-κB), and uromodulin, as compared to a control standard or the expression of the biomarkers in a control sample, wherein differential expression of the biomarkers in the sample as compared to the control standard or the expression of the biomarkers in a control sample indicates presence of breast cancer in the subject.

According to the disclosure, a biomarker for use is selected from a protein listed in Figure 5, or a gene or transcript that corresponds to a protein listed in Figure 5. In embodiments according to the disclosure, the biomarker is a protein, or gene thereof, that is shown as being differentially expressed in Figure 5 comparing cancer positive samples to cancer negative samples. In embodiments according to the disclosure, the biomarker is a protein, or gene thereof, that is shown in Figure 5 as having increased expression in cancer (second and third columns from the right of the table) as compared to a cancer negative sample (first column from the right of the table).

In various embodiments, once a cancer is detected in a subject, the subject may be treated for the cancer.

In order to facilitate review of the various disclosed embodiments, the following explanations of specific terms are provided.

Amplification of a nucleic acid molecule refers to methods used to increase the number of copies of a nucleic acid molecule, such as a uromodulin, MMP9, NF-κB, or MMP2 nucleic acid molecule. The resulting products can be referred to as amplicons or amplification products. Methods of amplifying nucleic acid molecules are known in the art, and include MDA, PCR (such as RT-PCR and qRT-PCR), DOP-PCR, RCA, T7/Primase-dependent amplification, SDA, 3SR, NASBA, and LAMP, among others.

A nucleic acid molecule is said to be "complementary" with another nucleic acid molecule if the two molecules share a sufficient number of complementary nucleotides to form a stable duplex or triplex when the strands bind (hybridize) to each other, for example by forming Watson-Crick, Hoogsteen or reverse Hoogsteen base pairs. Stable binding occurs when a nucleic acid molecule (*e.g*., nucleic acid probe or primer) remains detectably bound to a target nucleic acid sequence (*e.g*., an MMP9, NF-κB, uromodulin, or MMP2 target nucleic acid sequence) under the required conditions.

Complementarity is the degree to which bases in one nucleic acid molecule (*e.g.,* nucleic acid probe or primer) base pair with the bases in a second nucleic acid molecule (*e.g.,* target nucleic acid sequence). Complementarity is conveniently described by percentage, that is, the proportion of nucleotides that form base pairs between two molecules or within a specific region or domain of two molecules. For example, if 10 nucleotides of a 15 contiguous nucleotide region of a nucleic acid probe or primer form base pairs with a target nucleic acid molecule, that region of the probe or primer is said to have 66.67% complementarity to the target nucleic acid molecule.

In the present disclosure, "sufficient complementarity" means that a sufficient number of base pairs exist between one nucleic acid molecule or region thereof (such as a region of a probe or primer) and a target nucleic acid sequence (*e.g.*, an MMP9, NF-κB, uromodulin or MMP2 nucleic acid sequence) to achieve detectable binding.

Detection means to determine if an agent (*e.g.*, a nucleic acid molecule or protein) or interaction (*e.g.*, binding between two proteins, between a protein and a nucleic acid, or between two nucleic acid molecules) is present or absent. In some examples this can further include quantification. In particular examples, an emission signal from a label is detected. Detection can be in bulk, so that a macroscopic number of molecules can be observed simultaneously. Detection can also include identification of signals from single molecules using microscopy and such techniques as total internal reflection to reduce background noise.

For example, use of an antibody specific for a particular protein or biomarker (*e.g.,* MMP9, NF-κB, uromodulin or MMP2) permits detection of the protein or protein-protein interaction in a sample, such as a sample containing breast cancer tissue. In another example, use of a probe or primer specific for a particular gene (*e.g.,* MMP9, NF-κB, uromodulin or MMP2) permits detection of the of the desired nucleic acid molecule in a sample, such as a sample containing breast cancer tissue.

A nucleic acid sequence is differentially expressed when the amount of one or more of its expression products (*e.g.,* transcript (*e.g.,* mRNA) and/or protein) is higher or lower in one tissue (or cell) type as compared to another tissue (or cell) type.

A nucleic acid (*e.g*., genomic DNA, cDNA, or RNA) sequence that comprises coding sequences necessary for the production of a polypeptide, precursor, or RNA *(e.g.,* mRNA) is referred to as a gene. The polypeptide can be encoded by a full-length coding sequence or by any portion of the coding sequence so long as the desired activity or functional properties (*e.g*., enzymatic activity, ligand binding, signal transduction, immunogenicity, and the like) of the full-length or fragment is/are retained. The term also encompasses the coding region of a structural gene and the sequences located adjacent to the coding region on both the 5' and 3' ends for a distance of about 1 kb or more on either end such that the gene corresponds to the full-length mRNA. Sequences located 5' of the coding region and present on the mRNA are referred to as 5' untranslated sequences. Sequences located 3' or downstream of the coding region and present on the mRNA are referred to as 3' untranslated sequences. The gene as present in (or isolated from) a genome contains the coding regions ("exons") interrupted with non-coding sequences termed "introns." Introns are absent in the processed RNA (e.g., mRNA) transcript.

Gene expression is a multi-step process involving converting genetic information encoded in a genome and intervening nucleic acid sequences (*e.g*., mRNA) into a polypeptide. The genomic sequence of a gene is "transcribed" to produce RNA (*e.g.,* mRNA, also referred to as a transcript). mRNA is "translated" to produce a corresponding protein. Gene expression can be regulated at many stages in the process. Increased or decreased gene expression can be detected by an increase or decrease, respectively, in any gene expression product (*i.e.,* microRNA and/or mRNA and/or protein). Increased or decreased gene expression can also be a result of genomic alterations, such as an amplification or deletion, respectively, of the region of the genome including the subject gene sequence.

MicroRNAs (miRNA) are single-stranded RNA molecules, which regulate gene expression. miRNAs are encoded by genes from whose DNA they are transcribed but miRNAs are not translated into protein; instead each primary transcript (a pri-miRNA) is processed into a short stem-loop structure called a pre-miRNA and finally into a functional miRNA. Mature miRNA molecules are either fully or partially complementary to one or more messenger RNA (mRNA) molecules, and their main function is to down-regulate gene expression. MicroRNAs can be encoded by independent genes, but also be processed (via the enzyme Dicer) from a variety of different RNA species, including introns, 3' UTRs of mRNAs, long noncoding RNAs, snoRNAs and transposons. One skilled in the art, would appreciate that a biomarker of the invention may include a microRNA, for example, one that is known to be associated with a gene, protein or mRNA biomarker of the present invention (*i.e.,* a microRNA associated with expression of metalloproteinase 9 (MMP9), metalloproteinase 2 (MMP2), uromodulin, and nuclear factor kappa-light-chain-enhancer of activated B cells (NF-κB)).

In various embodiments according to the disclosure, microRNA biomarkers may include one or more of mir-10, mir-21, mir-155, mir-373, mir-30b, mir-126, mir-17p and mir-335. In one embodiment according to the disclosure, differential expression of mir-155 is utilized as a biomarker alone, or in combination with another microRNA or biomarker as described herein. According to one embodiment of the disclosure, increased expression of mir-155 is utilized as a biomarker alone, or in combination with another microRNA or biomarker as described herein.

In various embodiments according to the disclosure, increased expression indicative of breast cancer is detected for mi-155, metalloproteinase 9 (MMP9) and metalloproteinase 2 (MMP2) and nuclear factor kappa-light-chain-enhancer of activated B cells (NF-κB). In various embodiments according to the disclosure, increased expression indicative of breast cancer is detected for mi-155 and nuclear factor kappa-light-chain-enhancer of activated B cells (NF-κB). In various embodiments according to the disclosure, decreased expression indicative of breast cancer is detected for MMP2, MMP9, uromodulin, and IgG. In various embodiments according to the disclosure, decreased expression indicative of breast cancer is detected for uromodulin, and IgG. In various embodiments according to the disclosure, increased expression indicative of breast cancer is detected for mi-155 and nuclear factor kappa-light-chain-enhancer of activated B cells (NF-κB) and decreased expression indicative of breast cancer is detected for uromodulin, and IgG. In various embodiments according to the disclosure, differential expression is detected in all of mi-155, ADAM metallopeptidase domain 12 (ADAM12), metalloproteinase 9 (MMP9), metalloproteinase 12 (MMP12), metalloproteinase 2 (MMP2), uromodulin, IgG and nuclear factor kappa-light-chain-enhancer of activated B cells (NF-κB).

A label is an agent capable of detection, for example by spectrophotometry, flow cytometry, or microscopy. For example, one or more labels can be attached to an antibody, thereby permitting detection of a target biomarker. Furthermore, one or more labels can be attached to a nucleic acid molecule, thereby permitting detection of a target nucleic acid molecule (such as a biomarker DNA or RNA). Exemplary labels include radioactive isotopes, fluorophores, chromophores, ligands, chemiluminescent agents, enzymes, and combinations thereof.

Specific binding is the particular interaction between one binding partner (such as a gene-specific probe or protein-specific antibody) and another binding partner (such as a target of a gene-specific probe or protein-specific antibody). Such interaction is mediated by one or, typically, more non-covalent bonds between the binding partners (or, often, between a specific region or portion of each binding partner). In contrast to non-specific binding sites, specific binding sites are saturable. Accordingly, one exemplary way to characterize specific binding is by a specific binding curve. A specific binding curve shows, for example, the amount of one binding partner (the first binding partner) bound to a fixed amount of the other binding partner as a function of the first binding partner concentration. As the first binding partner concentration increases under these conditions, the amount of the first binding partner bound will saturate. In another contrast to non-specific binding sites, specific binding partners involved in a direct association with each other *(e.g.,* a probe-mRNA or antibody-protein interaction) can be competitively removed (or displaced) from such association by excess amounts of either specific binding partner. Such competition assays (or displacement assays) are very well known in the art.

As used herein, the term "sample" refers to any sample suitable for the methods provided by the present invention. The sample may be any sample that includes biomarkers suitable for detection. Sources of samples may include urine, whole blood, bone marrow, pleural fluid, peritoneal fluid, central spinal fluid, saliva and bronchial washes. In one aspect, the sample is a urine sample. A sample may be obtained and processed using well known and routine clinical methods. In various embodiments, the initial sample volume may be less than about 25 µl, 50 µl, 75 µl, 100 µl, 125 µl, 150 µl, 175 µl, 200 µl, 225 µl, 250 µl, 300 µl, 400 µl, 500 µl, 750 µl, 1 ml, 2 ml, 3 ml, 4 ml, 5 ml, 6 ml, 7 ml, 8 ml, 9 ml or greater than about 10 ml.

The term "cancer" as used herein, includes a variety of cancer types which are well known in the art, such as solid tumors. Cancers may include, but are not limited to, the following organs or systems: brain, cardiac, lung, gastrointestinal, genitourinary tract, liver, bone, nervous system, gynecological, hematologic, skin, breast, and adrenal glands. Additional types of cancer cells include gliomas (Schwannoma, glioblastoma, astrocytoma), neuroblastoma, pheochromocytoma, paraganlioma, meningioma, adrenalcortical carcinoma, medulloblastoma, rhabdomyoscarcoma, kidney cancer, vascular cancer of various types, osteoblastic osteocarcinoma, prostate cancer, ovarian cancer, uterine leiomyomas, salivary gland cancer, choroid plexus carcinoma, mammary cancer, pancreatic cancer, colon cancer, and megakaryoblastic leukemia; and skin cancers including malignant melanoma, basal cell carcinoma, squamous cell carcinoma, Karposi's sarcoma, moles dysplastic nevi, lipoma, angioma, dermatofibroma, keloids, sarcomas such as fibrosarcoma or hemangiosarcoma, and melanoma.

In some methods, expression of the gene(s) of interest is (are) measured in test (*i.e.,* cancer patient sample) and control samples relative to a value obtained for a housekeeping gene (*e.g.,* one or more of GAPDH (glyceraldehyde 3-phosphate dehydrogenase), SDHA (succinate dehydrogenase), HPRT1 (hypoxanthine phosphoribosyl transferase 1), HBS1L (HBS1-like protein), β-actin, and AHSP (alpha haemoglobin stabilizing protein)) in each sample to produce normalized test and control values; then, the normalized value of the test sample is compared to the normalized value of the control sample to obtain the relative expression of the gene(s) of interest (*e.g*., increased or decreased expression).

An increase or decrease in gene expression may mean, for example, that the expression of a particular gene expression product (*e.g.,* microRNA, transcript (*e.g.,* mRNA) or protein) in the test sample is at least about 1%, at least about 2%, at least about 5%, at least about 10%, at least about 15%, at least about 20%, at least about 25%, at least about 30%, at least about 50%, at least about 75%, at least about 100%, at least about 150%, or at least about 200% higher or lower as compared to the applicable control (*e.g*., standard value or control sample). Alternatively, relative expression (i.e., increase or decrease) may be in terms of fold difference; for example, the expression of a particular gene expression product (*e.g.,* microRNA, transcript (*e.g.,* mRNA) or protein) in the test sample may be at least about 2 fold, at least about 3 fold, at least about 4 fold, at least about 5 fold, at least about 8 fold, at least about 10 fold, at least about 20 fold, at least about 50 fold, at least about 100 fold, or at least about 200 fold times higher or lower as compared to the applicable control (*e.g*., standard value or control sample).

In some embodiments where protein expression as determined by immunohistochemistry is used as a measure of gene expression, scoring of protein expression may be semi-quantitative; for example, with protein expression levels recorded as 0, 1, 2, or 3 (including, in some instances plus (or minus) values at each level, *e.g.,* 1+, 2+, 3+) with 0 being substantially no detectable protein expression and 3 (or 3+) being the highest detected protein expression. In such methods, an increase or decrease in the corresponding gene expression is measured as a difference in the score as compared the applicable control (*e.g*., standard value or control sample); that is, a score of 3+ in a test sample as compared to a score of 0 for the control represents increased gene expression in the test sample, and a score of 0 in a test sample as compared to a score of 3+ for the control represents decreased gene expression in the test sample.

Disclosed herein is a kit comprising the above-noted panel of biomarkers. In one embodiment of the disclosure, there is provided a kit comprising the panel for detecting biomarkers of solid state cancer, wherein the biomarkers comprise at least two of ADAM12, MMP9, NF-κB, MMP12, MMP2, Hyaluronic Acid and instructions for use. The kit may further include one or more biomarkers corresponding to a protein or gene as set forth in Figure 5, especially those shown to exhibit increased expression in cancer.

The term "about," as used herein, is intended to qualify the numerical values which it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

### Determining Gene Expression Level

Gene expression levels may be determined in a disclosed method using any technique known in the art. Exemplary techniques include, for example, methods based on hybridization analysis of polynucleotides (*e.g*., genomic nucleic acid sequences and/or transcripts (*e.g*., mRNA)), methods based on sequencing of polynucleotides, methods based on detecting proteins (*e.g*., immunohistochemistry and proteomics-based methods).

Assay types that are useful to perform biomarker assays include, for example, immunochemical staining of cells and tissues, flow cytometry, enzyme linked immunosorbant assays (ELISA), and immunoprecipitation. Immunoassays can be performed as a sandwich assay or a competitive assay, in a lateral flow format or a comparable equivalent. It should be evident that when the biomarker is, for example, a protein, any assay that is capable of specifically and sensitively detecting the presence and amount of that protein in a sample can be used in the practice of the present invention based on well-known protein assay principles.

The biomarker assays described herein can be adapted to be performed by lay users without a laboratory. The users may be health care professionals in point-of-care facilities or lay consumers in field conditions. The devices may have multiple embodiments including single-use devices, simple reusable devices and computerized biomonitors. The single-use devices, similar to over-the-counter lateral flow assays for pregnancy, enable subjective multi-biomarker assays to be performed. Simple reusable devices also enable objective biomarker assays that provide a refined or enhanced indication of solid state cancer mass, and may also enable remote data processing.

Gene expression levels also can be determined by quantification of a microRNA or gene transcript (e.g., mRNA). Commonly used methods known in the art for the quantification of mRNA expression in a sample include, without limitation, northern blotting and in situ hybridization; RNAse protection assays; and PCR-based methods, such as reverse transcription polymerase chain reaction (RT-PCR) and real time quantitative PCR (also referred to as qRT-PCR). Alternatively, antibodies may be employed that can recognize specific duplexes, including DNA duplexes, RNA duplexes, and DNA-RNA hybrid duplexes, or DNA-protein duplexes. Representative methods for sequencing-based gene expression analysis include Serial Analysis of Gene Expression (SAGE), and gene expression analysis by massively parallel signature sequencing (MPSS).

Some method embodiments involving the determination of mRNA levels utilize RNA (*e.g.,* total RNA) isolated from a target sample, such a prostate cancer tissue sample. General methods for RNA (*e.g.,* total RNA) isolation are well known in the art and are disclosed in standard textbooks of molecular biology.

Differential gene expression also can be determined using microarray techniques. In these methods, specific binding partners, such as probes (including cDNAs or oligonucleotides) specific for RNAs of interest or antibodies specific for proteins of interest are plated, or arrayed, on a microchip substrate. The microarray is contacted with a sample containing one or more targets (*e.g*., microRNA, mRNA or protein) for one or more of the specific binding partners on the microarray. The arrayed specific binding partners form specific detectable interactions (*e.g*., hybridized or specifically bind to) their cognate targets in the sample of interest.

In some examples, differential gene expression is determined using in situ hybridization techniques, such as fluorescence in situ hybridization (FISH) or chromogen in situ hybridization (CISH). In these methods, specific binding partners, such as probes labeled with a fluorophore or chromogen specific for a target cDNA, microRNA or mRNA (*e.g.,* a biomarker cDNA or mRNA molecule or microRNA molecule) is contacted with a sample, such as a prostate cancer sample mounted on a substrate (*e.g.*, glass slide). The specific binding partners form specific detectable interactions (*e.g*., hybridized to) their cognate targets in the sample. For example, hybridization between the probes and the target nucleic acid can be detected, for example by detecting a label associated with the probe. In some examples, microscopy, such as fluorescence microscopy, is used.

Immunohistochemistry (IHC) is one exemplary technique useful for detecting protein expression products in the disclosed methods. Antibodies (*e.g*., monoclonal and/or polyclonal antibodies) specific for each protein expression marker are used to detect expression. The antibodies can be detected by direct labeling of the antibodies themselves, for example, with radioactive labels, fluorescent labels, hapten labels such as, biotin, or an enzyme such as horseradish peroxidase or alkaline phosphatase. Alternatively, unlabeled primary antibody is used in conjunction with a labeled secondary antibody, comprising antisera, polyclonal antisera or a monoclonal antibody specific for the primary antibody. IHC protocols and kits are well known in the art and are commercially available.

Further disclosed is that the biomarker assay can be provided in a kit, which allows for more convenient laboratory-based biomarker analysis. The kits may include a plurality of components including reagents, supplies, written instructions, and/or software. The kits may have a plurality of embodiments including laboratory kits and mail-in kits.

Laboratory biomarker assay kits may enable tests for individual and/or multi-biomarker in a laboratory. The kits may have a plurality of embodiments based on applications and methods for biomarker detection. The kits may also be designed for use with urine samples. Kit components can include: (1) sampling supplies and instructions; may include sample collectors and storage containers, sample processing tools, fixatives and user instructions; (2) controls; may be biological, such as urine, or synthetic samples of tissues or biofluids with baseline and elevated biomarker levels; and (3) biomarker-binding molecules including antibodies, aptamers, receptors, or other specific binding partners.

Biomarker-specific binding molecules may be provided as pre-made, ready-to-use reagents for detecting individual or combined biomarkers. The reagents may be optimized for tissue-specific applications. Alternatively, biomarker-specific binding molecules may be provided as concentrated reagents with suggested working concentrations for different applications. A pair of biomarker-specific binding molecules may be provided to enable double-positive biomarker recognition. Conjugated biomarker-specific binding molecules may also be provided, such as biomarker-specific binding molecules conjugated to biotin, fluorescent dyes or quantum dots.

The kits can include secondary reagents. Secondary reagents may be antibodies, enzymes, labels, or chemicals and may enable a complete biomarker panel assay.

Exemplary kits can include at least one means for detection of one or more of the disclosed genes or gene products (such as, at least two, at least three, at least four, or at least five detection means). In some examples, such kits can further include at least one means for detection of one or more (*e.g.,* one to three) housekeeping genes or proteins. Detection means can include, without limitation, a nucleic acid probe specific for a genomic sequence including a disclosed gene, a nucleic acid probe specific for a transcript (*e.g*., mRNA) encoded by a disclosed gene, a pair of primers for specific amplification of a disclose gene (*e.g*., genomic sequence or cDNA sequence of such gene), an antibody or antibody fragment specific for a protein encoded by a disclosed gene.

According to the disclosure, in some kit embodiments, the primary detection means (*e.g*., nucleic acid probe, nucleic acid primer, or antibody) can be directly labeled, *e.g*., with a fluorophore, chromophore, or enzyme capable of producing a detectable product (such as alkaline phosphates, horseradish peroxidase and others commonly known in the art). Other kit embodiments will include secondary detection means; such as secondary antibodies (*e.g*., goat anti-rabbit antibodies, rabbit anti-mouse antibodies, anti-hapten antibodies) or non-antibody hapten-binding molecules (*e.g*., avidin or streptavidin). In some such instances, the secondary detection means will be directly labeled with a detectable moiety. In other instances, the secondary (or higher order) antibody will be conjugated to a hapten (such as biotin, DNP, and/or FITC), which is detectable by a detectably labeled cognate hapten binding molecule (e.g., streptavidin (SA) horseradish peroxidase, SA alkaline phosphatase, and/or SA QDot^{™}). Some kit embodiments may include colorimetric reagents (*e.g.*, DAB, and/or AEC) in suitable containers to be used in concert with primary or secondary (or higher order) detection means (*e.g*., antibodies) that are labeled with enzymes for the development of such colorimetric reagents.

According to the disclosure, in some embodiments, a kit includes positive or negative control samples, such as a cell line or tissue known to express or not express a particular biomarker.

According to the disclosure, in some embodiments, a kit includes instructional materials disclosing, for example, means of use of a probe or antibody that specifically binds a disclosed gene or its expression product (*e.g*., microRNA, mRNA or protein), or means of use for a particular primer or probe. The instructional materials may be written, in an electronic form (*e.g.,* computer diskette or compact disk) or may be visual (*e.g.,* video files). The kits may also include additional components to facilitate the particular application for which the kit is designed. Thus, for example, the kit can include buffers and other reagents routinely used for the practice of a particular disclosed method. Such kits and appropriate contents are well known to those of skill in the art.

According to the disclosure, certain kit embodiments can include a carrier means, such as a box, a bag, a satchel, plastic carton (such as molded plastic or other clear packaging), wrapper (such as, a sealed or sealable plastic, paper, or metallic wrapper), or other container. In some examples, kit components will be enclosed in a single packaging unit, such as a box or other container, which packaging unit may have compartments into which one or more components of the kit can be placed. In other examples, a kit includes a one or more containers, for instance vials, tubes, and the like that can retain, for example, one or more biological samples to be tested.

Other kit embodiments according to the disclosure, include, for instance, syringes, cotton swabs, or latex gloves, which may be useful for handling, collecting and/or processing a biological sample. Kits may also optionally contain implements useful for moving a biological sample from one location to another, including, for example, droppers, syringes, and the like. Still other kit embodiments may include disposal means for discarding used or no longer needed items (such as subject samples). Such disposal means can include, without limitation, containers that are capable of containing leakage from discarded materials, such as plastic, metal or other impermeable bags, boxes or containers.

The kits can further include software. Software may include a training video that may provide additional support including demonstration of biomarker assays, examples of results, or educational materials for performing biomarker assays according to the disclosure.

Mail-in biomarker assay sample collection kits enable sample collection and shipment to a remote laboratory for testing. The remote laboratory may perform biomarker assays using assay kits, and provide test results to the user. Potential users include lay consumers, and professional users in the field or point-of-care facility. Mail-in tests may have a plurality of embodiments based on samples and applications. The samples may include body fluids, such as urine, secretions and tissues. Components can include:
(1) Supplies and instructions for collecting and fixing samples to enable mailing and subsequent laboratory analysis of biomarkers. The supplies may include sample collectors, sample processing tools and supplies, fixatives, storage containers. The supplies may enable preparation of stabilized samples of whole biofluids and tissues; or cellular spreads made from biofluids. Technical support via telephone and a website.
(2) Mailing supplies to enable sending samples to a remote laboratory that performs biomarker tests. The supplies may be a pre-addressed regular mail envelope.
(3) Results provided by mail. The mail may be a letter, an email, information posted on a website. The website may have health tips and links to health care and product providers. The links may be advertisements.

Conveniently, assays for use in the methods of the invention can be configured into a test kit for use in a laboratory setting. Such kits may include a disposable module for sample uptake and reagent storage (refills sold separately), and a re-usable module for signal detection and result display that may involve optical and electronic components. Real-time results (1-3 minutes) may be obtained from test strip assay formats. Simple readout of results, *e.g*., indicative of a biomarker concentration above baseline via, for example, a colormetric or optical reflectance difference observed are particularly effective means for communicating assay results.

The assay may alternatively be formatted for performance in a sampling strip (a plastic microscopy slide), a collection cup, a plastic spatula, a small pouch with fixative (alcohol), instructions for making and fixing a smear, a mailing envelope/packaging addressed to testing company. Fixed slides can be send by regular mail (biomarkers are stable). The testing company processes the slide and sends results back via self-addressed envelope and/or the results are posted on the testing company's website (via personalized access code).

The following examples are provided to further illustrate the embodiments of the present invention, but are not intended to limit the scope of the invention. While they are typical of those that might be used, other procedures, methodologies, or techniques known to those skilled in the art may alternatively be used.

### EXAMPLE 1

### EXPRESSION PROFILES IN BREAST CANCER

Protein expression analysis was performed to determine expression profiles in breast cancer samples and normal samples. Protein expression profiles were analyzed for two breast cancer positive samples and one breast cancer negative sample.

Results are tabulated in Figure 5 which sets forth expression levels of various proteins in the three samples that were analyzed. Breast cancer negative samples are samples 1 and 4 corresponding to columns 1 and 2 of results. The breast cancer positive sample is sample 5 and shown in the third column on the right of the table (last column on the right). Differential expression of various proteins is evident between positive and negative samples. Any proteins that are differentially expressed between the positive and negative samples may be useful as a biomarker, especially those which are determined to have increased expression in cancer as compared to control. Proteins not present in a sample are indicated by a zero (0) in the results columns while probability of presence of a protein is shown as a positive numerical value with shading.

### EXAMPLE 2

### EXPRESSION PROFILES IN BREAST CANCER

Previous studies have evaluated miRNA expression level in breast cancer. Hafez et al. (Asian Pac J Cancer Prev. 13(2):591-8 (2012)) investigate eight miRNAs and eight metastatic-related genes in 40 breast cancer samples and their adjacent non-neoplastic tissues. The expression levels of each miRNA relative to U6 RNA was determined. Also, miRNA expression profiles of the BC and their corresponding ANT were evaluated.

The breast cancer patients showed an up-regulation in miRNAs (mir-155, mir-10, mir-21 and mir-373) with an upregulation in MMP2, MMp9 and VEGF genes. Down regulation was determined for mir-17p, mir-126, mir-335, mir-30b and also TIMP3, TMP1 and PDCD4 genes in the cancer tissue compared to the adjacent non-neoplastic tissues. Mir -10b, mir -21, mir-155 and mir373 and the metastatic genes MMP2, MMP9 and VEGF were significantly associated with an increase in tumor size (P<0.05).

The present example evaluates use of mir-155 alone, or in combination with one or more biomarkers selected from ADAM metallopeptidase domain 12 (ADAM12), metalloproteinase 9 (MMP9), metalloproteinase 12 (MMP12), metalloproteinase 2 (MMP2), nuclear factor kappa-light-chain-enhancer of activated B cells (NF-κB), uromodulin and IgG. Detection of differential expression in cancer as compared to normal at an increased level of specificity and/or sensitivity as compared to prior studies is expected. For example, detection of an increase in expression of mi-155 and decrease of expression of uromodulin and IgG is unexpected and likely will allow detection of breast cancer at an increased level of specificity and/or sensitivity as compared with prior methods. Further this will allow a simplified screening assay that utilizes fewer biomarkers and that may be performed in any laboratory setting.

## Claims

1. A method of detecting breast cancer in a subject, comprising determining, in a sample from the subject, the expression level of biomarkers of solid tumor microenvironment potential or solid state tumor mass potential consisting of metalloproteinase 9 (MMP9), metalloproteinase 2 (MMP2), nuclear factor kappa-light-chain-enhancer of activated B cells (NF-κB), and uromodulin, as compared to a control standard or the expression of the biomarkers in a control sample, wherein differential expression of the biomarkers in the sample as compared to the control standard or the expression of the biomarkers in a control sample indicates presence of breast cancer in the subject.

2. The method of claim 1, wherein
(a) determining the expression level comprises measuring the level of an expression product of the biomarker;
(b) the expression product is protein or mRNA; or
(c) the subject is a human.

3. The method of claim 1, wherein
(a) the expression level is increased or decreased;
(b) the expression level is determined by ELISA; or
(c) expression level measurements are quantitative.

4. The method of claim 1, wherein the sample is a urine sample.

## Patentansprüche

1. Ein Verfahren zum Nachweis von Brustkrebs in einem Subjekt, umfassend das Bestimmen des Expressionslevels von Biomarkern des Mikroumgebungspotentials eines soliden Tumors oder des Massenpotentials eines Solid-State-Tumors bestehend aus Metalloproteinase 9 (MMP9), Metalloproteinase 2 (MMP2), Nuklear Factor Kappa-Light-Chain-Enhancer von aktivierten B-Zellen (NF-κB) und Uromodulin, in einer Probe des Subjekts, im Vergleich zu einem Kontrollstandard oder der Expression der Biomarker in einer Kontrollprobe, wobei die unterschiedliche Expression der Biomarker in der Probe, verglichen mit dem Kontrollstandard oder der Expression der Biomarker in einer Kontrollprobe, das Vorliegen von Brustkrebs in dem Subjekt anzeigt.

2. Das Verfahren nach Anspruch 1, wobei
(a) das Bestimmen des Expressionslevels das Messen des Spiegels eines Expressionsproduktes des Biomarkers umfasst;
(b) das Expressionsprodukt ein Protein oder mRNA ist; oder
(c) das Subjekt ein Mensch ist.

3. Das Verfahren nach Anspruch 1, wobei
(a) das Expressionslevel erhöht oder verringert ist;
(b) das Expressionslevel mittels ELISA bestimmt wird; oder
(c) die Messungen des Expressionslevels quantitativ sind.

4. Das Verfahren nach Anspruch 1, wobei die Probe eine Urinprobe ist.

## Revendications

1. Procédé de détection d'un cancer du sein chez un sujet, comprenant la détermination, dans un échantillon provenant du sujet, du niveau d'expression de biomarqueurs du potentiel de microenvironnement de tumeur solide ou du potentiel de masse de tumeur à l'état solide, consistant en la métalloprotéinase 9 (MMP9), la métalloprotéinase 2 (MMP2), le facteur nucléaire kappa-light-chain-enhancer de cellules B activées (NF-κB), et l'uromoduline, par rapport à une norme de contrôle ou à l'expression des biomarqueurs dans un échantillon de contrôle, dans lequel l'expression différentielle des biomarqueurs dans l'échantillon par rapport à la norme de contrôle ou à l'expression des biomarqueurs dans un échantillon de contrôle indique la présence d'un cancer du sein chez le sujet.

2. Procédé selon la revendication 1, dans lequel
(a) la détermination du niveau d'expression comprend la mesure du niveau d'un produit d'expression du biomarqueur ;
(b) le produit d'expression est une protéine ou un ARNm ; ou
(c) le sujet est un humain.

3. Procédé selon la revendication 1, dans lequel
(a) le niveau d'expression est augmenté ou diminué ;
(b) le niveau d'expression est déterminé par ELISA ; ou
(c) les mesures du niveau d'expression sont quantitatives.

4. Procédé selon la revendication 1, dans lequel l'échantillon est un échantillon d'urine.
